# EUROPEAN PATENT APPLICATION

(11) **EP 4 447 229 A1**
(43) Date of publication of application: **16.10.2024**
(21) Application number: 24166068.7
(22) Date of filing: 25.03.2024
(51) Int. Cl.: H01R 11/28, H01R 13/20, H01R 13/6581, H01R 13/6592, H01R 9/05, H01R 101/00

(54) **ANTI-NOISE ELECTRODE CONNECTOR AND METHOD FOR MANUFACTURING THE SAME**

(30) Priority: 11.04.2023 TW 112113389
(71) Applicant: New V-Key Technology Co., Ltd., New Taipei City 23544 (TW)
(72) Inventor: Ono, Kohei, Tokyo 188-0011 (JP); Hsieh, Cheng-Change, 23544 New Taipei City (TW)
(74) Representative: Prinz & Partner mbB

(57) **Abstract**

An anti-noise electrode connector and a method for manufacturing the same are provided. The anti-noise electrode connector includes an electrode snap socket (20), a wire, an inner insulating layer (30), an anti-noise layer (40), and an outer insulating layer (50). The wire (10) includes a conductive core wire (11), an insulator (11C), a metal shielding layer (12) disposed at an outer periphery of the insulator (11C), and a cable jacket (13). The insulator (11C) covers the conductive core wire (11). The cable jacket (13) covers the metal shielding layer (12). The metal shielding layer (12) has an exposed section (12E), the conductive core wire (11) has a connection section (11E) that is exposed, and the connection section (11E) extends beyond the metal shielding layer (12) of the wire (10). The connection section (11E) is connected to a top surface of the electrode snap socket (20). The inner insulating layer (30) covers the electrode snap socket (20). The anti-noise layer (40) covers the inner insulating layer (30) and the exposed section (12E) of the metal shielding layer (12).

## Description

### FIELD OF THE INVENTION

The present invention relates to an anti-noise electrode connector, and more particularly to an electrode connector having an anti-noise function, so as to be isolated from external interferences and used for, for example, medical purposes (such as for detecting physiological signals of the human body).

### BACKGROUND OF THE INVENTION

A medical electrode patch can be used to detect various electrical signals of the human body, such as being used for electrocardiograms. However, the electrical signals of the heart are weak, and are easily subjected to external interferences (e.g., static electricity) during a detection process.

Therefore, how to enhance an anti-noise effect of an electrode connector through improvements in structural design, so as to overcome the above-mentioned problem, has become one of the important issues to be solved in the related art.

### SUMMARY OF THE INVENTION

In response to the above-referenced technical inadequacy, the present invention provides an anti-noise electrode connector and a method for manufacturing the same, so that an electrode connector can have an anti-noise function to avoid external interferences.

In order to solve the above-mentioned problem, one of the technical aspects adopted by the present invention is to provide an anti-noise electrode connector, which includes a wire, an electrode snap socket, an inner insulating layer, an anti-noise layer, and an outer insulating layer. The wire includes a conductive core wire, an insulator, a metal shielding layer, and a cable jacket. The insulator covers the conductive core wire, the metal shielding layer is disposed at an outer periphery of the insulator, and the cable jacket covers the metal shielding layer. The metal shielding layer has an exposed section, and the exposed section extends beyond the cable jacket. The conductive core wire has a connection section that is exposed, and the connection section extends beyond the insulator. The connection section is connected to a top surface of the electrode snap socket. The inner insulating layer covers the electrode snap socket. The anti-noise layer covers the inner insulating layer and the exposed section of the metal shielding layer. The outer insulating layer covers the anti-noise layer, and is connected to the cable jacket of the wire.

In order to solve the above-mentioned problem, another one of the technical aspects adopted by the present invention is to provide a method for manufacturing an anti-noise electrode connector. The method includes: providing an electrode snap socket; providing a wire, in which the wire includes a conductive core wire, an insulator, a metal shielding layer, and a cable jacket, the insulator covers the conductive core wire, the metal shielding layer is disposed at an outer periphery of the insulator, and the cable jacket covers the metal shielding layer; forming an exposed section by configuring the metal shielding layer to extend beyond the cable jacket; forming a connection section that is exposed by configuring the conductive core wire to extend beyond the metal shielding layer; connecting the connection section to a top surface of the electrode snap socket; forming an inner insulating layer to cover the electrode snap socket; forming an anti-noise layer to cover the inner insulating layer and the exposed section of the metal shielding layer; and forming an outer insulating layer to cover the anti-noise layer, in which the outer insulating layer is connected to the cable jacket of the wire.

Therefore, in the anti-noise electrode connector and the method for manufacturing the same provided by the present invention, an anti-noise effect of the electrode connector can be achieved by having the connection section connected to the top surface of the electrode snap socket and the anti-noise layer cover the exposed section of the metal shielding layer. In this way, electrostatic interferences of the surrounding environment can be avoided.

These and other aspects of the present disclosure will become apparent from the following description of the embodiment taken in conjunction with the following drawings and their captions, although variations and modifications therein may be affected without departing from the spirit and scope of the novel concepts of the disclosure.

### BRIEF DESCRIPTION OF THE DRAWINGS

The described embodiments may be better understood by reference to the following description and the accompanying drawings, in which:
FIG. 1 is a schematic perspective view of an anti-noise electrode connector according to a first embodiment of the present invention;
FIG. 2 is a schematic exploded view of the anti-noise electrode connector according to the first embodiment of the present invention;
FIG. 3 is another schematic exploded view of the anti-noise electrode connector according to the first embodiment of the present invention;
FIG. 4 is a schematic cross-sectional view of the anti-noise electrode connector according to the first embodiment of the present invention; and
FIG. 5 is a schematic cross-sectional view of the anti-noise electrode connector according to a second embodiment of the present invention.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

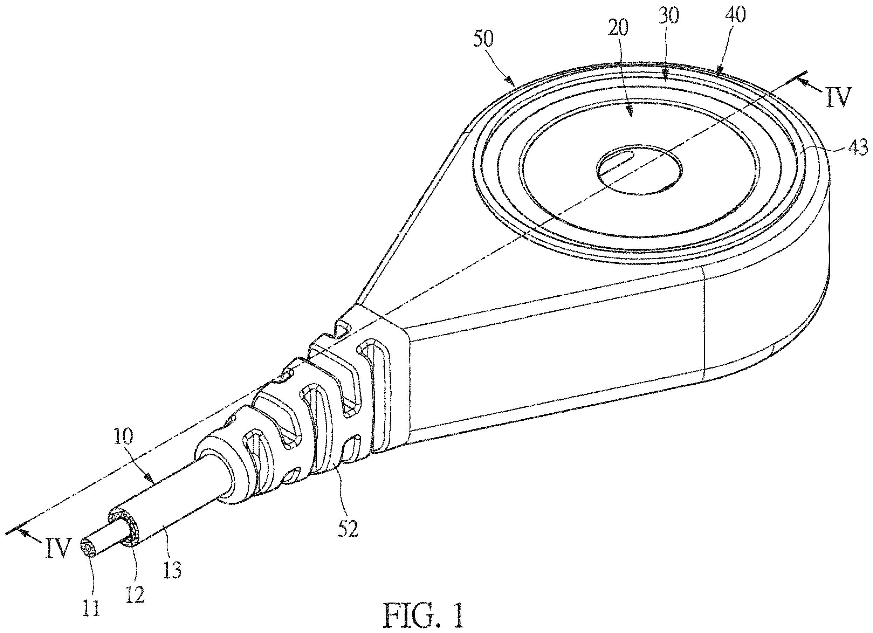

Referring to FIG. 1 to FIG. 4, a first embodiment of the present invention provides an anti-noise electrode connector, which includes a wire 10, an electrode snap socket 20, an inner insulating layer 30, an anti-noise layer 40, and an outer insulating layer 50. The electrode snap socket 20 can be fastened to a conductive stud 92 of a conductive patch 9. For example, the anti-noise electrode connector of the present invention is applicable to the conductive patch 9 used for electrocardiography (ECG), electromyography (EMG), and electroencephalography (EEG), but the present invention is not limited thereto. The conductive patch 9 has a gel region 94 that is disposed below the conductive stud 92 and an outer surface layer 93. The conductive stud 92 is electrically connected to the gel region 94.

The wire 10 is an anti-interference wire or cable. Specifically, the wire 10 includes a conductive core wire 11, an insulator 11C, a metal shielding layer 12, and a cable jacket 13. The insulator 11C covers the conductive core wire 11, and the insulator 11C can be, for example, a plastic insulating layer. The metal shielding layer 12 is disposed at an outer periphery of the insulator 11C, and the cable jacket 13 covers the metal shielding layer 12.

The metal shielding layer 12 can be formed by braiding or twisting multiple strands of metal wires (e.g., copper wires), or can be a metal film (e.g., an aluminum/Mylar foil) that wraps around the insulator 11C. In the present embodiment, the metal shielding layer 12 has an exposed section 12E, which is a portion that is not covered by the cable jacket 13. The exposed section 12E extends beyond the cable jacket 13. The conductive core wire 11 has a connection section 11E that is exposed, and the connection section 11E extends beyond the metal shielding layer 12. The connection section 11E is connected to a top surface of the electrode snap socket 20. The connection section 11E is welded to the top surface of the electrode snap socket 20, but the present invention is not limited thereto. Other ways of forming the connection can be, for example, by soldering, spot welding, or crimping.

Preferably, the wire 10 of the present embodiment further includes a second shielding layer 12C that has an isolation function. The second shielding layer 12C can be another conductive shielding layer, such as an aluminum foil or a carbon tube. The second shielding layer 12C is preferably the carbon tube, or can be referred to as a conductive carbon-based inner shielding layer. The conductive carbon-based inner shielding layer covers the insulator 11C, and the metal shielding layer 12 covers the conductive carbon-based inner shielding layer, so as to further enhance an anti-noise effect. Specifically, the second shielding layer 12C is disposed between the metal shielding layer 12 and the insulator 11C, and extends beyond the metal shielding layer 12. A portion of the second shielding layer 12C is exposed from a surface of the insulator 11C, and is in contact with the anti-noise layer 40. However, the present invention is not limited to a wire having two shielding layers. The wire of the present invention can have only one shielding layer (e.g., only having the metal shielding layer 12).

In the present embodiment, the metal wires of the metal shielding layer 12 extend beyond the cable jacket 13, and are reversely bent to form the exposed section 12E. In other words, the metal wires are reversely bent in a direction away from the electrode snap socket 20, and the exposed section 12E covers a front end portion of the cable jacket 13. However, the present invention is not limited thereto.

The anti-noise electrode connector of the present invention further includes a metal ring 15. The exposed section 12E of the metal shielding layer 12 is clamped by the metal ring 15. Specifically, the exposed section 12E of the metal shielding layer 12 is fixed to the cable jacket 13 by the metal ring 15 in a crimping manner. In the present embodiment, the metal ring 15 is annular-shaped, and a width of the metal ring 15 is less than or equal to a length of the exposed section 12E. In other words, the metal ring 15 does not completely cover the exposed section 12E.

The inner insulating layer 30 covers the electrode snap socket 20. In the present embodiment, the inner insulating layer 30 is formed by insert molding of a plastic insulating material. However, the present invention is not limited thereto.

One feature of the present embodiment is that the anti-noise layer 40 is provided. The anti-noise layer 40, which can be called a shielding layer, covers the inner insulating layer 30, the exposed section 12E of the metal shielding layer 12, and the metal ring 15. The material of the anti-noise layer 40 is a conductive material, such as a metallic material or a conductive polymer. The conductive polymer can be, for example, polyvinyl chloride (PVC), polypropylene (PP), acrylonitrile butadiene styrene (ABS), nylon, and polycarbonate (PC). The anti-noise layer 40 is formed on an outer layer of the inner insulating layer 30 by injection molding, or more specifically, by insert molding. However, the present invention is not limited to said material and said manufacturing method. For example, the anti-noise layer 40 can also be obtained by spraying or printing with conductive ink, bonding with metal or a conductive tape, or assembling other conductive materials.

Furthermore, the anti-noise layer 40 of the present embodiment extends beyond a bottom surface of the electrode snap socket 20 (as shown in FIG. 1), so as to form an annular-shaped flange 43. Preferably, the flange 43 has a range that can completely cover the gel region 94 of the conductive patch 9. The gel region 94 is a portion that actually contacts the skin for retrieval of signals. In other words, a radius of the flange 43 is greater than or equal to a radius of the gel region 94. However, the present invention is not limited thereto. In the present invention, the flange 43 can also be omitted. An orthogonal projection area (or a coverage area) of the anti-noise layer 40 is greater than or equal to an area of the gel region 94 of the conductive patch 9. Accordingly, the anti-noise layer 40 allows the electrode snap socket 20 to be insulated from electrostatic interferences of the surrounding environment, so as to achieve an anti-noise function.

In the present embodiment, the anti-noise layer 40 also covers the metal ring 15. Through the metal ring 15, positions and an area in contact with the anti-noise layer 40 can be increased. In addition, a protruding structure of the metal ring 15 can also increase a bonding strength between the anti-noise layer 40 and the wire 10. During the process of forming the anti-noise layer 40 by insert molding, the metal shielding layer 12 of the wire 10 can be firmly fixed by the metal ring 15.

The outer insulating layer 50 covers the anti-noise layer 40 and the cable jacket 13 of the wire 10. The outer insulating layer 50 can be formed by injection molding of an insulating material. More specifically, the outer insulating layer 50 of the present embodiment can be formed by insert molding techniques, but the present invention is not limited thereto.

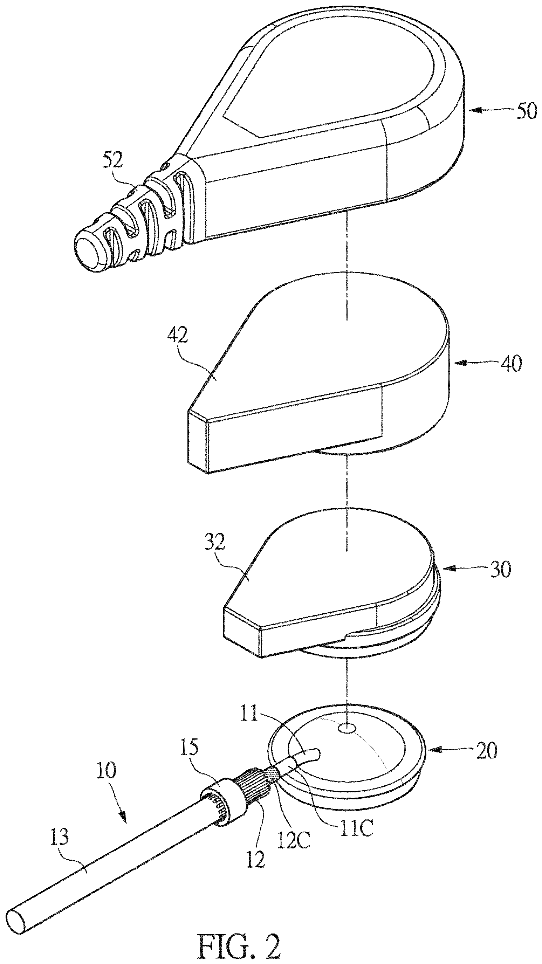

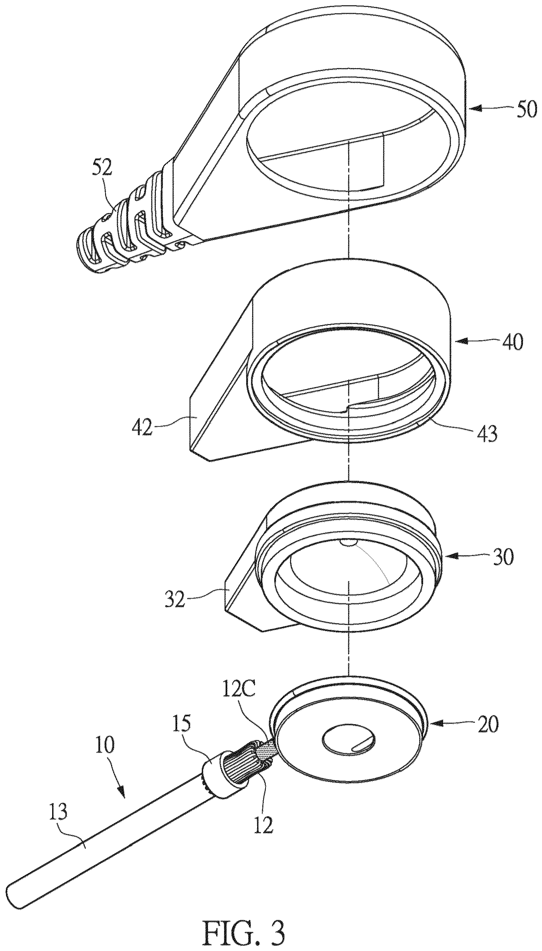

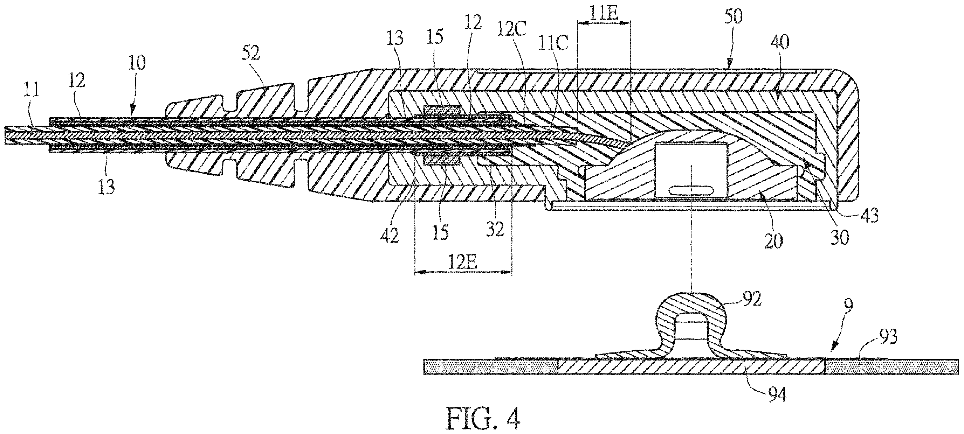

Reference is made to FIG. 2 and FIG. 3. In the present embodiment, from a top view, the inner insulating layer 30, the anti-noise layer 40, and the outer insulating layer 50 are drop-shaped. Referring to FIG. 4, the inner insulating layer 30 has a first extension portion 32, the anti-noise layer 40 has a second extension portion 42, and the outer insulating layer 50 has a third extension portion 52. The first extension portion 32 covers a small portion of the exposed section 12E that is reversely bent. The second extension portion 42 covers a substantial portion of the exposed section 12E, and an area of the second extension portion 42 is greater than an area of the first extension portion 32. The second extension portion 42 is disposed at an outer periphery of the electrode snap socket 20, and covers the metal ring 15. The third extension portion 52 covers an outer periphery of the wire 10. Through this configuration, a shielding structure of the present embodiment is very stable during an injection molding process, and two ends of the exposed section 12E can be firmly fixed, thereby ensuring complete connection and conduction between the metal shielding layer 12 and the anti-noise layer 40.

The present invention further provides a method for manufacturing the anti-noise electrode connector. As shown in FIG. 1 to FIG. 4, the method at least includes the following steps: providing the electrode snap socket 20; providing the wire 10, in which the wire 10 includes the conductive core wire 11, the insulator 11C, the metal shielding layer 12, and the cable jacket 13, the insulator 11C covers the conductive core wire 11, the metal shielding layer 12 is disposed at the outer periphery of the insulator 11C, and the cable jacket 13 covers the metal shielding layer 12; forming the exposed section 12E by configuring the metal shielding layer 12 to extend beyond the cable jacket 13; forming the connection section 11E that is exposed by configuring the conductive core wire 11 to extend beyond the metal shielding layer 12; connecting the connection section 11E to the top surface of the electrode snap socket 20; forming the inner insulating layer 30 to cover the electrode snap socket 20; forming the anti-noise layer 40 to cover the inner insulating layer 30 and the exposed section 12E of the metal shielding layer 12; and forming the outer insulating layer 50 to cover the anti-noise layer 40, in which the outer insulating layer 50 is connected to the cable jacket 13 of the wire 10.

The formation steps in the above-mentioned method of the present invention can be insert molding techniques, and the merit thereof is that the anti-noise layer 40 can be formed between the inner insulating layer 30 and the outer insulating layer 50 during the injection molding process. In this way, the stability and durability of an integral electrode connector can be enhanced, thereby reducing the mechanical space required for assembling a housing and assembly components that may be detached after long use. However, the above-mentioned embodiment is merely an exemplary embodiment of the present invention, and the present invention is not limited to this way of formation.

### [Second Embodiment]

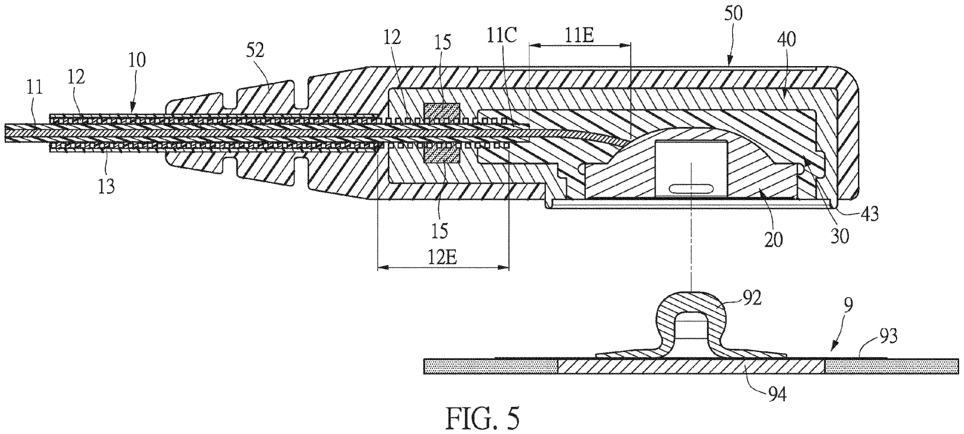

As shown in FIG. 5, the difference between the present embodiment and the above-mentioned embodiment is that the exposed section 12E of the metal shielding layer 12 is not reversely bent. The exposed section 12E can be multiple strands of metal wires, a metal layer, a carbon tube, or a combination thereof. The exposed section 12E of the metal shielding layer 12 passes through the second extension portion 42 of the anti-noise layer 40 from the outer insulating layer 50, and extends into the inner insulating layer 30. It should be noted that the metal ring 15 can be omitted.

### [Beneficial Effects of the Embodiments]

In conclusion, in the anti-noise electrode connector and the method for manufacturing the same provided by the present invention, through the technical features of the connection section being connected to the top surface of the electrode snap socket, the inner insulating layer covering the electrode snap socket, the anti-noise layer covering the inner insulating layer and the exposed section of the metal shielding layer, etc., a shielding effect of the electrode connector can be enhanced, and electrostatic interferences of the surrounding environment can be avoided.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope.

## Claims

1. An anti-noise electrode connector, **characterized by** comprising:
an electrode snap socket (20);
a wire (10), wherein the wire (10) includes a conductive core wire (11), an insulator (11C), a metal shielding layer (12), and a cable jacket (13), the insulator (11C) covers the conductive core wire (11), the metal shielding layer (12) is disposed at an outer periphery of the insulator (11C), the cable jacket (13) covers the metal shielding layer (12), the metal shielding layer (12) has an exposed section (12E), the exposed section (12E) extends beyond the cable jacket (13), the conductive core wire (11) has a connection section (11E) that is exposed, and the connection section (11E) extends beyond the insulator (11C); wherein the connection section (11E) is connected to a top surface of the electrode snap socket (20);
an inner insulating layer (30) covering the electrode snap socket (20);
an anti-noise layer (40) covering the inner insulating layer (30) and the exposed section (12E) of the metal shielding layer (12); and
an outer insulating layer (50) covering the anti-noise layer (40), wherein the outer insulating layer (50) is connected to the cable jacket (13) of the wire (10).

2. The anti-noise electrode connector according to claim 1, wherein the anti-noise layer (40) has a flange (43), and the flange (43) extends beyond a bottom surface of the electrode snap socket (20).

3. The anti-noise electrode connector according to claim 1 or 2, wherein the anti-noise layer (40) is a conductive polymer, and is formed on an outer layer of the inner insulating layer (30) by insert molding.

4. The anti-noise electrode connector according to any of the preceding claims, wherein the metal shielding layer (12) of the wire (10) includes multiple strands of metal wires, the metal wires extend beyond the cable jacket (13) and are reversely bent to form the exposed section (12E), and the exposed section (12E) covers a front end portion of the cable jacket (13).

5. The anti-noise electrode connector according to any of the preceding claims, further comprising a metal ring (15), wherein the exposed section (12E) of the metal shielding layer (12) is fixed to the cable jacket (13) by the metal ring (15) in a crimping manner, and the anti-noise layer (40) is in contact with the metal ring (15).

6. The anti-noise electrode connector according to claim 5, wherein the metal ring (15) is annular-shaped, and a width of the metal ring (15) is less than or equal to a length of the exposed section (12E).

7. The anti-noise electrode connector according to any of the preceding claims, wherein the inner insulating layer (30) has a first extension portion (32), the first extension portion (32) covers a portion of the exposed section (12E) that is reversely bent, the anti-noise layer (40) has a second extension portion, an area of the second extension portion is greater than an area of the first extension portion (32), and the second extension portion covers the metal ring (15).

8. The anti-noise electrode connector according to claim 7, wherein the exposed section (12E) of the metal shielding layer (12) passes through the second extension portion of the anti-noise layer (40) from the outer insulating layer (50), and extends into the inner insulating layer (30).

9. The anti-noise electrode connector according to any of the preceding claims, wherein the wire (10) further includes a second shielding layer (12C), the second shielding layer (12C) is disposed between the metal shielding layer (12) and the insulator (11C), and the second shielding layer (12C) extends beyond the metal shielding layer (12); wherein a portion of the second shielding layer (12C) is exposed from a surface of the insulator (11C), and is in contact with the anti-noise layer (40).

10. The anti-noise electrode connector according to any of the preceding claims, wherein an orthogonal projection area of the anti-noise layer (40) is greater than or equal to an area of a gel region (94) of a conductive patch (9), and the conductive patch (9) is configured to be fastened to the electrode snap socket (20).

11. A method for manufacturing an anti-noise electrode connector, **characterized by** comprising:
providing an electrode snap socket (20);
providing a wire, wherein the wire (10) includes a conductive core wire (11), an insulator (11C), a metal shielding layer (12), and a cable jacket (13), the insulator (11C) covers the conductive core wire (11), the metal shielding layer (12) is disposed at an outer periphery of the insulator (11C), and the cable jacket (13) covers the metal shielding layer (12);
forming an exposed section (12E) by configuring the metal shielding layer (12) to extend beyond the cable jacket (13);
forming a connection section (11E) that is exposed by configuring the conductive core wire (11) to extend beyond the metal shielding layer (12);
connecting the connection section (11E) to a top surface of the electrode snap socket (20);
forming an inner insulating layer (30) to cover the electrode snap socket (20);
forming an anti-noise layer (40) to cover the inner insulating layer (30) and the exposed section (12E) of the metal shielding layer (12); and
forming an outer insulating layer (50) to cover the anti-noise layer (40), wherein the outer insulating layer (50) is connected to the cable jacket (13) of the wire (10).
